(19) Europäisches Patentamt
European Patent Office
Office européen des brevets

(11) **EP 4 063 009 A1**

(12) **EUROPEAN PATENT APPLICATION**
published in accordance with Art. 153(4) EPC

(43) Date of publication:
**28.09.2022 Bulletin 2022/39**

(21) Application number: **20889847.8**

(22) Date of filing: **18.11.2020**

(51) International Patent Classification (IPC):
**B01J 8/24** (2006.01)          **C07C 253/26** (2006.01)
**B01J 8/44** (2006.01)

(52) Cooperative Patent Classification (CPC):
**B01J 8/18; B01J 8/24; B01J 8/44; C07C 253/26;
C07C 255/08**

(86) International application number:
**PCT/CN2020/129819**

(87) International publication number:
**WO 2021/098728 (27.05.2021 Gazette 2021/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.11.2019 CN 201911153609**

(71) Applicants:
• **China Petroleum & Chemical Corporation
Beijing 100728 (CN)**

• **Shanghai Research Institute of Petrochemical
Technology, SINOPEC
Shanghai 201208 (CN)**

(72) Inventors:
• **ZHAO, Le
Shanghai 201208 (CN)**
• **WU, Lianghua
Shanghai 201208 (CN)**

(74) Representative: **karo IP
karo IP Patentanwälte
Kahlhöfer Rößler Kreuels PartG mbB
Postfach 32 01 02
40416 Düsseldorf (DE)**

(54) **GAS DISTRIBUTION PLATE, FLUIDIZATION DEVICE, AND REACTION METHOD**

(57)     Disclosed is a gas distribution plate, comprising a metal plate, central openings and peripheral openings, wherein a ratio D1/D1' of the aperture diameter D1 (expressed in a unit of mm) of the central opening to the aperture diameter D1' (expressed in a unit of mm) of the peripheral opening satisfies the relation of $1.10 \geq D1/D1' > 1.00$. A fluidizing device comprising the gas distribution plate and application of the fluidizing device in an oxidation or ammoxidation reaction process are also disclosed. The gas distribution plate has an advantage of uniform gas distribution.

**Fig. 2A**

EP 4 063 009 A1

**Fig. 2B**

**Description**

**Technical Field**

The present invention relates to a gas distribution plate, and particularly to an air distribution plate. The invention also relates to a fluidizing device comprising the gas distribution plate and application thereof in an oxidation process or an ammoxidation process.

**Background Art**

Acrylonitrile is produced by propylene ammoxidation all over the world, and in the production process, there is a certain requirement on the ratio between the staring materials, for example, the ratio of ammonia to propylene is 1-1.5, and the ratio of air to propylene is 8.5-10.5. As a matter of fact, such a ratio is just in the explosion limit range of propylene-ammonia, and in the production process, the three starting materials are not introduced into a reaction bed after mixing but are separately introduced into the bed, in which the mixture of propylene and ammonia is introduced into the bed through one distributor, and the air is introduced into the bed through another distributor. Propylene, ammonia and air are subjected to ammoxidation reaction preferentially under the action of a catalyst, so that the explosive concentration range of the feed gas may be avoided.

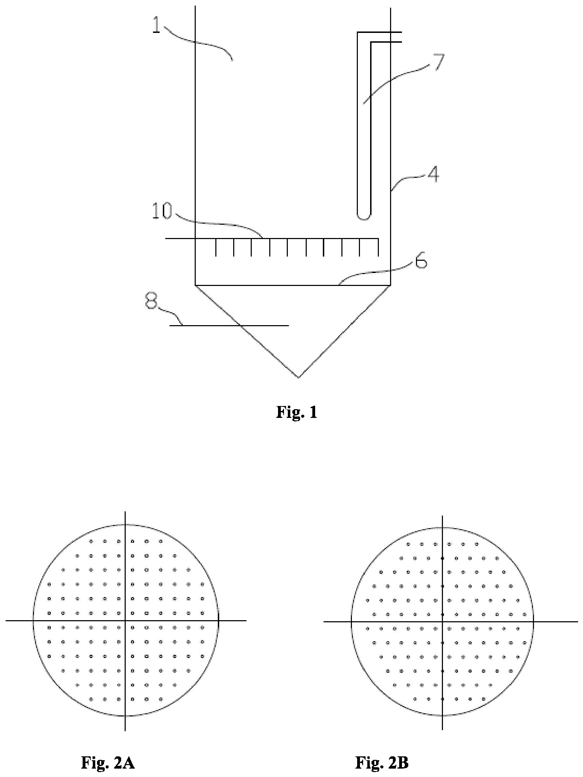

As shown in Fig. 1, in an ammoxidation fluidized bed reactor 1, the respective components are, successively, an air inlet 8, a gas distribution plate (also referred to as an air distribution plate) 6, a propylene-ammonia feed distributor 10, a reactor wall 4, and a heat removal water pipe 7. Air is introduced into the reactor through the inlet 8, passed through the gas distribution plate 6, uniformly mixed with the propylene-ammonia feed gas introduced into the reactor through the feed distributor 10, and then passed into the catalyst bed, so that an ammoxidation reaction can be conducted.

The gas distribution plate is one of the important inner components of the acrylonitrile fluidized bed reactor, and the quality of the design of the gas distribution plate has a direct impact on the fluidization quality of the fluidized bed and the operation performance of the reactor. A typical gas distribution plate for acrylonitrile fluidized bed is in the form of a perforated plate provided with short pipes, and specifically, the gas distribution plate comprises a continuous metal plate and gas distribution plate nozzles. Air flows into the nozzles through the orifices at the end of the nozzles and then flows into the bed through the nozzles of the air distribution plate. The feed gas and air are guided by each nozzle of the gas distribution plate and dispersed into the bed from bottom to top.

For an acrylonitrile fluidized bed reactor, the pressure drop of the distributor may be influenced by the change in the structure of the gas distribution plate, and in order to ensure a uniform distribution of the gas flow and avoid serious non-uniformity caused by certain fluctuation or load reduction, the distributor is normally required to have enough pressure drop.

The document "Study on Capacity Expansion and Modification of Acrylonitrile Plant (Contemporary Chemical Industry, volume 34, No. 5)" discloses that the pressure drop of the gas distribution plate is about 60% of the pressure drop of the bed, which is believed to result in a uniform gas distribution and a velocity of passing through the openings that is not so high as to cause severe erosion of the catalyst.

GB 1265770A discloses a fluidized bed reactor, in which an influence of the wall effect can be eliminated by fluidizing the catalyst deposited near the reactor wall with a fluid by means of an annular distribution pipe arranged on the distributor plate in a position close to the reactor wall, concentric with the distributor plate.

**Disclosure of the Invention**

The inventors of the present invention have found that, as the production scale of acrylonitrile increases, the reactor diameter also increases, and if the pressure drop of the gas distribution plate described in the document" Study on Capacity Expansion and Modification of Acrylonitrile Plant" is still used, the quality of fluidization near the reactor wall will be deteriorated due to the wall boundary effect, and the reaction results will be affected finally. Therefore, the pressure drop design parameters of existing gas distribution plate are not suitable for the requirement of the design of large-scale fluidized bed reactor.

The inventor of the present invention have also found that, in fluidized bed reactors widely used at present, the feed gas and air are separately fed, and an uniform mixing of them is realized by means of the feed distributor and the gas distribution plate, and thus if the wall effect is eliminated by blowing the air in a direction opposite to the gas distribution plate using the distribution pipe disclosed in GB1265770A, the dispersion effect of the gas distribution plate will inevitably be adversely affected, and in turn the mixing of the air and the feed gas will be affected. Moreover, the separate arrangement of the distribution pipe not only increases the equipment cost, but also complicates the internal structure of the reactor, which may adversely affect the fluidization quality.

The inventors of the present invention have also found that the acrylonitrile fluidized bed is an in which a bubble phase and a granular phase are present. In aggregative fluidization, the gas moves upwards through the bed mainly in the form of bubbles, and the movement of the bubbles in the bed plays an important role in heat transfer, mass transfer and chemical reaction. After the gas passes through the distributor, bubbles can be formed. Bubbles are formed at the moment that the gas is introduced into the bed through the openings of the gas distribution plate, the size and the shape of the bubbles are related to the characteristics of catalyst particles, the characteristics of the gas, the diameter of the openings and the flow velocity of gas passing through the openings, the content of catalyst in the bubble phase is relative low and may account for about 1% of the total amount of the catalyst in the bed, and an exchange of substance between the gas and entrained catalyst in the bubble phase and the catalyst and entrained gas in the particle phase may occur, so that the gas in the particle phase may enter the bubbles, and meanwhile, a part of the gas in the bubbles may also penetrate through the boundaries of the bubbles and permeate into the catalyst particles. Small bubbles are more favorable for mass transfer than large bubbles.

The inventors of the present invention have also found that the initial bubble formation, which is related to the flow velocity of gas passing through the openings, at a position slightly above the gas distribution plate is critical to a good fluidization quality of the acrylonitrile fluidized bed reactor. A typical gas distribution plate for acrylonitrile fluidized bed is a perforated metal plate, and it requires a same and smooth velocity of gas passing through each opening in order to achieve a same fluidization quality per unit cross section at a position slightly above the gas distribution plate.

The inventors of the present invention have also found that, because the fluid velocity between the position near the reactor wall and the center of the reactor has a certain gradient due to the influence of objective factors such as viscosity and wall friction during the movement of the fluid, the fluid near the reactor wall is in a relatively static state, and the catalyst near the reactor wall is in a downward settling state relative to the catalyst at the center of the reactor. In other words, the catalyst at the center of the reactor has a better fluidization quality than the catalyst near the reactor wall. The fluidization quality near the reactor wall may be deteriorated due to the wall boundary effect, and consequently the propylene ammoxidation may not reach the optimal reaction state, and in turn the propylene conversion rate in the near-reactor wall area may be reduced, thereby affecting the reaction result of the whole reactor. This result cannot satisfy the demand for the enlargement of the reactor. Therefore, there is a need in the art to develop a gas distributor plate that may not reduce the pressure drop of the gas distributor plate, but can reduce the influence of the wall effect on the fluidization quality near the reactor wall, suppress the decrease of the fluidization quality of the catalyst near the reactor wall, and meet the requirement of fast and uniform mixing at a the position near the reactor wall of the gases from the distribution plate/distributor.

The inventors of the present invention have found through a great deal of research and experiments that the fluidization quality near the reactor wall can be improved by properly reducing the inner diameter of the nozzle near the reactor wall to increase the fluid velocity of the air passing through the opening, so that the conversion rate of the starting material at corresponding position can be increased, and in turn the yield of acrylonitrile of the whole reaction unit can be increased correspondingly.

The present invention has been accomplished based on these findings.

Specifically, the present invention relates to the following aspects:

1. A gas distribution plate (particularly an air distribution plate or an air distribution plate for an ammoxidation fluidized bed reactor) comprising a metal plate (particularly a flat metal plate), openings provided in a central region of the metal plate (referred to as central openings) and openings provided in a peripheral region of the metal plate (referred to as peripheral openings), wherein a ratio $D1/D1'$ of an aperture diameter $D1$ (expressed in a unit of mm) of the central opening to an aperture diameter $D1'$ (expressed in a unit of mm) of the peripheral opening satisfies the relation $1.10 \geq D1/D1' > 1.00$, preferably $1.08 \geq D1/D1' > 1.00$, and more preferably $1.06 \geq D1/D1' \geq 1.01$.

2. The gas distribution plate according to any of the preceding or subsequent aspects, wherein the aperture diameter $D1$ of each of the central openings is the same as or different from each other (preferably the same as each other) and is independently 16-60 mm, preferably 20-56 mm, more preferably 22-52 mm, and/or the aperture diameter $D1'$ of each of the peripheral openings is the same as or different from each other (preferably the same as each other) and is independently 15-58 mm, preferably 19-54 mm, more preferably 21-50 mm.

3. The gas distribution plate according to any of the preceding or subsequent aspects, wherein the number of central openings is from 16 to 100 (preferably from 17 to 64, more preferably from 18 to 44) per square meter of the central region, and/or the number of peripheral openings is from 2 to 50 (preferably from 3 to 44, more preferably from 4 to 25) per square meter of the peripheral region, and/or the number of central openings is from 70% to 99% (preferably from 75% to 98%, more preferably from 80% to 95%) of the total number of openings in the metal plate.

4. The gas distribution plate according to any of the preceding or subsequent aspects, wherein the number of central openings per unit area of the central region is substantially the same.

5. The gas distribution plate according to any of the preceding or subsequent aspects, wherein the central openings and/or the peripheral openings are arranged substantially in the form of a square, an equilateral triangle, an equilateral rhombus, or concentric circles, preferably substantially in the form of a square or an equilateral triangle.

6. The gas distribution plate according to any of the preceding or subsequent aspects, wherein the distances between any two adjacent central openings are the same as or different from each other (preferably the same as each other), and are each independently 100-300 mm, preferably 125-285 mm, more preferably 150-270 mm, and/or the distances between any two adjacent peripheral openings are the same as or different from each other (preferably the same as each other), and are each independently 100-300 mm, preferably 125-285 mm, more preferably 150-270 mm.

7. The gas distribution plate according to any of the preceding or subsequent aspects, wherein the metal plate has a substantially circular shape, the circle having a diameter of 5 to 29 m, preferably 7 to 20 m, and a thickness of 5 to 40 mm, preferably 10 to 35 mm.

8. The gas distribution plate according to any of the preceding or subsequent aspects, where a straight-line distance between any point on the outer periphery of the metal plate and a central point of the metal plate is designated as R (particularly, a radius), a region surrounded by all points on the metal plate at a straight-line distance r from the central point is referred to as the central region, and a region between the central region and the outer periphery is referred to as the peripheral region, the value of r/R is 0.2 to 0.99, preferably 0.5 to 0.9, and more preferably 0.7 to 0.85.

9. The gas distribution plate according to any of the preceding or subsequent aspects, wherein at least one (preferably all) of the central openings have a nozzle (referred to as central nozzle), wherein the central nozzle is a hollow tube with a starting end of the central nozzle being inserted into the central opening, perpendicularly connected to the metal plate and coaxial with the central opening, and a tail end of the central nozzle having an orifice (referred to as central orifice), and/or at least one (preferably all) of the peripheral openings have a nozzle (referred to as peripheral nozzle), wherein the peripheral nozzle is a hollow tube with a starting end of the peripheral nozzle being inserted into the peripheral opening, perpendicularly connected to the metal plate and coaxial with the peripheral opening, and a tail end of the peripheral nozzle having an orifice (referred to as peripheral orifice).

10. The gas distribution plate according to any of the preceding or subsequent aspects, wherein the aperture diameter d of each of the central orifices is the same as or different from each other (preferably the same as each other) and is independently 5 to 20mm, preferably 7 to 18mm, and more preferably 10 to 16 mm, and/or the aperture diameter d' of each of the peripheral orifices is the same as or different from each other (preferably the same as each other) and is independently 5 to 20 mm, preferably 7 to 18 mm, and more preferably 10 to 16 mm, and/or the aperture diameter d of the central orifice is the same as or different from the aperture diameter d' of the peripheral orifice, and/or d/d' satisfies the relation $1.10 \geq d/d' \geq 1.00$ (preferably $1.04 \geq d/d' \geq 1.00$).

11. The gas distribution plate according to any of the preceding or subsequent aspects, wherein the central nozzles and/or the peripheral nozzles have an injection angle $\alpha$ of 2° to 20°, preferably 4° to 17°, more preferably 5° to 14°.

12. The gas distribution plate according to any of the preceding or subsequent aspects, wherein the length of the central nozzle and/or the peripheral nozzle is 80-300 mm, preferably 100-270 mm, and more preferably 120-240 mm.

13. The gas distribution plate according to any of the preceding or subsequent aspects, where the aperture diameter of the center orifice is designated as d (expressed in a unit of mm), the aperture diameter of the peripheral orifice is designated as d' (expressed in a unit of mm), the aperture diameter of the central opening is designated as D1 (expressed in a unit of mm), and the aperture diameter of the peripheral opening is designate as D1' (expressed in a unit of mm), (d'/D1')/(d/D1) $\geq$ 1, preferably (d'/D1')/(d/D1) = 1-1.25, (d'/D1')/(d/D1) = 1-1.20, or (d'/D1')/(d/D1) = 1.01-1.10.

14. The gas distribution plate according to any of the preceding or subsequent aspects, wherein the inner diameter D2 of each of the central nozzles is the same as or different from each other (preferably the same as each other) and is independently 6 to 50 mm, preferably 10 to 47 mm, more preferably 12 to 44 mm, and/or the inner diameter D2' of each of the peripheral nozzles is the same as or different from each other (preferably the same as each other) and is independently 5 to 48 mm, preferably 9 to 45 mm, more preferably 11 to 42 mm.

15. A gas distribution plate, comprising a metal plate, central openings provided in a central region of the metal plate, and peripheral openings provided in a peripheral region of the metal plate, wherein at least one (preferably all) of the central openings have a nozzle (referred to as central nozzle), wherein the central nozzle is a hollow tube with a starting end of the central nozzle being inserted into the central opening, perpendicularly connected to the metal plate and coaxial with the central opening, and a tail end of the central nozzle having an orifice (referred to as central orifice), and at least one (preferably all) of the peripheral openings have a nozzle (referred to as peripheral nozzle), wherein the peripheral nozzle is a hollow tube with a starting end of the peripheral nozzle being inserted into the peripheral opening, perpendicularly connected to the metal plate and coaxial with the peripheral opening, and a tail end of the peripheral nozzle having an orifice (referred to as peripheral orifice), and where an aperture diameter of the central orifice is designated as d (expressed in a unit of mm), an aperture diameter of the peripheral orifice is designated as d' (expressed in a unit of mm), an aperture diameter of the central opening is designated as D1 (expressed in a unit of mm), and an aperture diameter of the peripheral opening is designated as D1' (expressed in a unit of mm), (d'/D1')/(d/D1) $\geq$ 1, preferably (d'/D1')/(d/D1) = 1-1.25, (d'/D1')/(d/D1) = 1-1.20, or (d'/D1')/(d/D1) = 1.01-1.10.

16. A fluidizing device (preferably a fluidized bed reactor), comprising at least a housing, a fluidizing device chamber defined by the housing, and a gas distribution plate disposed in the fluidizing device chamber, wherein the gas distribution plate is a gas distribution plate according to any of the preceding or subsequent aspects.

17. The fluidizing device according to any of the preceding or subsequent aspects, wherein the fluidizing device chamber has a bed of solid particles (preferably catalyst particles), and wherein a pressure drop $\Delta Pd$ (expressed in a unit of MPa) of the gas distribution plate is 62-120%, preferably 65-115%, more preferably 68-110% of a pressure drop $\Delta Pb$ (expressed in a unit of MPa) of the bed of solid particles.

18. An oxidation or ammoxidation process, comprising the step of subjecting a feedstock (preferably a garbage or hydrocarbon feedstock, more preferably $C_{2-8}$ olefin or propylene) to oxidation or ammoxidation reaction with an oxidizing gas (preferably air or oxygen) to produce an oxidation or ammoxidation product (preferably propylene oxide or acrylonitrile) using a gas distribution plate according to any of the preceding or subsequent aspects as the oxidizing gas (preferably air or oxygen) distribution plate or in a fluidized bed reactor according to any of the preceding or subsequent aspects.

19. A method for distributing a gas, comprising the step of delivering the gas through a gas distribution plate according to any of the preceding or subsequent aspects from one side thereof to the other, wherein the flow velocity of gas passing through the peripheral opening is not less than the flow velocity of gas passing through the central opening.

## Brief Description of the Drawings

Fig. 1 is a schematic representation of a fluidized bed reactor of the present invention.
Fig. 2A and 2B are schematic diagrams of the arrangement of the openings of the gas distribution plate of the present invention.
Figs. 3 and 4 are schematic diagrams of the nozzle of the present invention.
Fig. 5 is a schematic diagram of the pressure monitoring of the gas distribution plate of the present invention.

## Description of the Reference Numerals:

1. Fluidized bed reactor
2. Orifice of gas distribution plate
3. Nozzle of gas distribution plate
4. Wall of fluidized bed reactor
5. Pressure measuring port above the distribution plate of fluidized bed reactor
6. Gas distribution plate
7. Heat removal water pipe
8. Air inlet of fluidized bed reactor
9. Pressure measuring port in the cone of fluidized bed reactor
10. Propylene-ammonia feeding distributor

## Technical effects

By using the gas distribution plate of the present invention, an effect of uniform gas distribution can be realized.
By using the fluidizing device of the present invention, a good fluidization quality can be achieved.
By using the gas distribution plate of the present invention, a maximum utilization rate of the catalyst can be obtained and a reduction of dead zones of the catalyst can be achieved.
By monitoring the pressure drop $\Delta Pd$ of the gas distribution plate of the present invention, the working state of the gas distribution plate can be monitored in real time.

## Detailed Description of the Invention

The present application will be illustrated in detail hereinbelow with reference to embodiments thereof, but it should be noted that the scope of the present application is not limited by those embodiments, but is defined by the appended claims.
All publications, patent applications, patents, and other references cited herein are incorporated by reference in their entirety. Unless defined otherwise, all technical and scientific terms used herein have the same meaning as commonly understood by those skilled in the art. In case of conflict, the contents described herein, including definitions, should prevail.

When a material, substance, method, step, device, component, or the like described herein is modified by an expression "commonly known to those skilled in the art", "known in the art" or the like, it is to be understood that said material, material, substance, method, step, device, component, or the like covers not only those conventionally used in the art at the time of filing the present application, but also those not commonly used at present but will become commonly known in the art to be suitable for a similar purpose.

In the context of the present specification, the term "flat plate" covers the case of a flat plate, as well as the case of a substantially flat plate, and the case that normally understood by those skilled in the art to be a flat plate.

In the context of the present application, the term "substantially" means that a deviation acceptable or considered reasonable to those skilled in the art, such as within $\pm$ 10%, within $\pm$ 5%, within $\pm$ 1%, within $\pm$ 0.5% or within $\pm$ 0.1%, is allowable to be present.

In the context of the present application, unless specifically stated otherwise, all percentages, parts, ratios, etc. are expressed by weight and all pressures given are gauge pressures.

In the context of the present application, any two or more embodiments of the present application may be arbitrarily combined, and the resulting technical solution forms a part of the initial disclosure of the present application and falls within the scope of the present application.

According to an embodiment of the present invention, it relates to a gas distribution plate. Here, as the gas distribution plate, an air distribution plate, especially an air distribution plate used in an ammonia oxidation fluidized bed reactor or an acrylonitrile fluidized bed reactor, may be particularly mentioned. As is known, the gas distribution plate is typically in the form of a perforated flat plate.

According to an embodiment of the present invention, the gas distribution plate comprises a metal plate, openings provided in a central region of the metal plate (referred to as central openings), and openings provided in a peripheral region of the metal plate (referred to as peripheral openings). These openings are through holes extending through the metal plate from its upper surface to its lower surface, which are provided for introducing a gas into the fluidizing device or the bed of solid particles. Particularly, a straight-line distance between any point on the outer periphery of the metal plate and a central point of the metal plate is designated as R, a region surrounded by all points on the metal plate having a straight-line distance r from the central point is referred to as the central region, and a region between the central region and the outer periphery is referred to as the peripheral region. Here, if the metal plate has a substantially circular shape, R is a radius of the metal plate and r is a radius of the central region.

It is known that, particularly for ammoxidation or acrylonitrile fluidized bed reactor applications, the metal sheet is a flat metal sheet. When a flat metal plate is used as the metal plate, it is possible to obtain a fluid velocity near the reactor wall that is not lower than a fluid velocity at the center of the reactor, that is, a flow velocity of gas passing through the peripheral opening (see below) that is not lower than a flow velocity of gas passing through the central opening (see below), whereby the fluidization quality of the catalyst near the reactor wall can be improved. Here, the velocity is typically characterized herein by a linear velocity, which may be in a range from about 6 m/s to about 25 m/s, although the present invention is not limited thereto. In addition, the flow velocity can be easily obtained by directly measuring the gas velocity at the outlet of the corresponding opening.

According to an embodiment of the present invention, a uniform distribution of gas or air can be achieved by delivering the gas through the gas distribution plate from one side thereof to the other. Here, as previously described, the flow velocity of gas passing through the peripheral opening is not lower than the flow velocity of gas passing through the central opening.

According to an embodiment of the present invention, the value of r/R is from 0.2 to 0.99, preferably from 0.5 to 0.9, more preferably from 0.7 to 0.85.

According to an embodiment of the present invention, the ratio D1/D1' of the aperture diameter D1 (expressed in a unit of mm) of the central opening to the aperture diameter D1' (expressed in a unit of mm) of the peripheral opening satisfies the relation 1.10 $\geq$ D1/D1' > 1.00, preferably 1.08 $\geq$ D1/D1' > 1.00, more preferably 1.06 $\geq$ D1/D1' $\geq$ 1.01.

According to an embodiment of the present invention, the aperture diameter D1 of each of the central openings is the same as or different from each other, preferably the same as each other, and is independently 16 to 60 mm, preferably 20 to 56 mm, more preferably 22 to 52 mm.

According to an embodiment of the present invention, the aperture diameter D1' of each of the peripheral openings is the same as or different from each other, preferably the same as each other, and is independently 15 to 58 mm, preferably 19 to 54 mm, more preferably 21 to 50 mm.

According to an embodiment of the present invention, the inventors of the present application have found through theoretical calculation and experimental verification on the initial bubbles that the number of central openings is 16 to 100 per square meter of the area of the central region, preferably 17 to 64 or 18 to 44 per square meter of the area of the central region. In order to achieve more uniform introduction of the gas, it is preferable that the central region is provided with the same number of central openings per unit cross sectional area.

According to an embodiment of the present invention, the number of central openings is 70-99%, preferably 75-98%, more preferably 80-95% of the total number of openings in the metal sheet.

According to an embodiment of the present invention, the inventors of the present application have found through theoretical calculation and experimental verification on the initial bubbles that the number of the peripheral openings is 2 to 50 per square meter of the area of the peripheral region, preferably 3 to 44 or 4 to 25 per square meter of the area of the peripheral region. In order to achieve more uniform introduction of the gas, it is preferable that the peripheral region is provided with the same number of peripheral openings per unit cross sectional area.

According to an embodiment of the present invention, the number of central openings per unit area of the central region is substantially the same.

According to an embodiment of the present invention, the arrangement of the central openings is not particularly limited, but is substantially in the form of a square, an equilateral triangle, an equilateral rhombus, or concentric circles, preferably substantially in the form of a square or an equilateral triangle, as shown in Figs. 2A and 2B.

According to an embodiment of the present invention, the arrangement of the peripheral openings is not particularly limited, but is substantially in the form of a square, an equilateral triangle, an equilateral rhombus, or concentric circles, preferably substantially in the form of a square or an equilateral triangle, as shown in Figs. 2A and 2B.

Taking an acrylonitrile fluidized bed reactor as an example, and referring to the square arrangement shown in Fig. 2A, the inventors of the present application have found that, if the space between openings is too much larger than the diameter of bubbles, the gas ejected from four openings is not sufficient to fluidize the catalyst at the center of the square while flowing upwards, and the non-fluidized catalyst, like a small fixed bed, will cause ammoxidation reaction of the feed gas on the catalyst surface and finally lose activity due to over-reduction. The catalyst in the center of the square does not participate in the ammoxidation reaction of propylene, and this results in a reduction in the utilization rate of the catalyst, an increase in the catalyst load of the reactor, and finally a negative impact on the reaction result. On the other hand, the space between openings should not be too small. Where the space between openings is smaller than the diameter of bubbles, coalescence of bubbles generated above adjacent openings may easily occur, and since the volume of a coalesced bubble is larger than the sum of the volumes of two bubbles before coalescence, more reaction gas may pass through the bed via bubble short circuit, and this results in a reduction in the chance of contact between the gas and catalyst particles, a reduction in the efficiency of mass transfer, and finally a deteriorated reaction performance as well. Thus, a suitable space between openings is desirable for providing a good initial fluidization condition in the fluidized bed, and it is preferable that the space between openings is slightly larger than the diameter of initial bubbles generated on the gas distribution plate.

According to an embodiment of the present invention, the inventors of the present application have found through theoretical calculation and experimental verification on the initial bubbles that the distances between any two adjacent central openings (i.e. the space between openings) are the same as or different from each other, preferably the same as each other, and are each independently 100-300 mm, preferably 125-285 mm, and more preferably 150-270 mm. Here, the space between openings is defined as a side length of the pattern of the arrangement of the openings.

According to an embodiment of the present invention, the inventors of the present application have found through theoretical calculation and experimental verification on the initial bubbles that the distances between any two adjacent peripheral openings (i.e. the space between openings) are the same as or different from each other, preferably the same as each other, and are each independently 100-300 mm, preferably 125-285 mm, and more preferably 150-270 mm. Here, the space between openings is defined as a side length of the pattern of the arrangement of the openings.

According to an embodiment of the present invention, the metal plate has a substantially circular shape, with the diameter of the circle being typically 5-29 m, preferably 7-20 m.

According to an embodiment of the present invention, the thickness of the metal plate is typically 5 to 40 mm, preferably 10 to 35 mm.

According to an embodiment of the present invention, at least one, preferably all, of the central openings have a nozzle (referred to as central nozzle). In addition, at least one (preferably all) of the peripheral openings have a nozzle (referred to as peripheral nozzle).

According to an embodiment of the present invention, the central nozzle is a hollow tube, and a starting end of the central nozzle is inserted into the central opening, perpendicularly connected to the metal plate and coaxial with the central opening. Preferably, the end of the central nozzle has an orifice (referred to as central orifice). In addition, the peripheral nozzle is a hollow tube, and a starting end of the peripheral nozzle is inserted into the peripheral opening, perpendicularly connected to the metal plate and coaxial with the peripheral opening. Preferably, the end of the peripheral nozzle has an orifice (referred to as peripheral orifice).

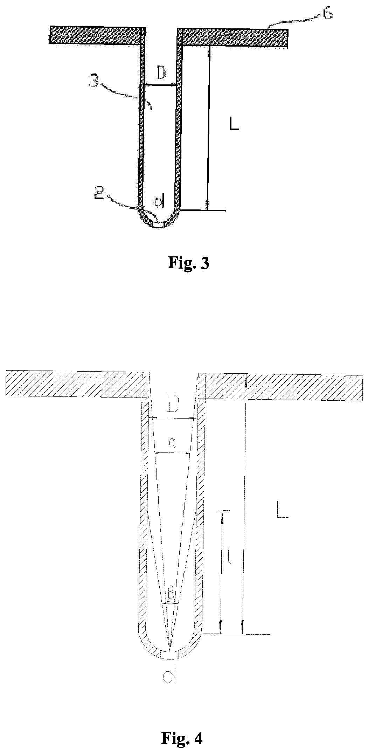

The inventors of the present invention have found that, in order to avoid erosion of solid particles such as catalyst by a gas of high velocity, the gas introduced into the interior of a fluidizing device such as a fluidized bed reactor from a gas inlet 8 is typically not allowed to pass through openings in a gas distribution plate directly, but instead nozzles 3 are provided on the metal plate of the gas distribution plate 6 as shown in Fig. 3. The plurality of nozzles 3 are disposed on the lower surface of the metal plate and correspond to the openings in the metal plate one by one. The upper end of the nozzle 3 is connected to an opening in the metal plate, the middle part as a main body of the nozzle is a cylindrical hollow metal tube, and the lower end of the nozzle is provided with an orifice 2. The nozzle is perpendicular to the metal plate, and the nozzle body, the orifice and the opening are coaxial. That is, a short pipe (i.e., nozzle) is used for connecting the opening and the orifice, and the opening, the orifice and the nozzle are coaxial. In addition, in order to achieve a flow rectification effect, the inner diameter D of the nozzle 3 is set to be larger than the diameter d of the orifice 2, and at the other end, the diameter of the opening is set to be the same as the outer diameter of the nozzle 3. As for the inner diameter D of the nozzle 3, it is typically set to be such that provides a velocity of the gas in the nozzle body in a range of 8 to 50m/s, preferably 12 to 40 m/s.

According to an embodiment of the present invention, the connection between the nozzle and the metal plate is not particularly limited, and a conventional connection such as welding or screwing may be used.

According to an embodiment of the present invention, the aperture diameter d of each of the central orifices is the same as or different from each other, preferably the same as each other, and is independently from 5 to 20 mm, preferably from 7 to 18 mm, more preferably from 10 to 16 mm.

According to an embodiment of the present invention, the aperture diameter d' of each of the peripheral orifices is the same as or different from each other, preferably the same as each other, and is independently from 5 to 20 mm, preferably from 7 to 18 mm, more preferably from 10 to 16 mm.

According to an embodiment of the present invention, the aperture diameter d of the central orifice is the same as or different from the aperture diameter d' of the peripheral orifice. Preferably, d/d' satisfies the relation $1.10 \geq d/d' \geq 1.00$, preferably $1.04 \geq d/d' \geq 1.00$.

The inventors of the present application have found through fluid mechanics research that, since the flow velocity and direction of the fluid change as the diameter changes in the process of the gas passing through the orifice to the nozzle, a backflow of a part of the fluid may be generated near the position with a change of diameter, which may cause a change of the fluid state at said position, such as a change of laminar motion into turbulent motion, and the fluid in the turbulent region has disordered and unstable motion state. The turbulent region gradually gets close to the pipe wall along the flowing direction of the fluid till the turbulent region is eliminated, so that the motion state of the fluid gradually turns into a stable laminar motion along the flowing direction. When viewed from the flow field of the fluid, in the region from the position with a change of diameter to the position where the turbulent region is eliminated, a certain boundary line exists between the turbulent-flow layer and the laminar-flow layer. As shown in Fig. 4, the length of the nozzle 3 from the position with a change of diameter to the upper surface of the gas distribution plate is designated as a nozzle length L, and the length of the nozzle 3 from the position with a change of diameter to the position where the turbulent region is eliminated is designated as a minimum nozzle length 1, then,

the injection angle $\alpha$ may be expressed in the following equation (1):

$$\alpha = 2\mathrm{acrtg}(D/2L) \quad (1)$$

the injection angle $\beta$ may be expressed in the following equation (2):

$$\beta = 2\mathrm{acrtg}(D/2l) \quad (2)$$

wherein D represents the inner diameter of the nozzle expressed in a unit of mm, and L represents the nozzle length expressed in a unit of mm, and 1 represents the minimum nozzle length expressed in a unit of mm.

According to the present invention, as can be seen from the above equations (1) and/or (2), the smaller the injection angle $\alpha$ and/or $\beta$, i.e., the smaller the ratio of the inner diameter D of the nozzle to the nozzle length L and/or 1, the longer the nozzle length L and/or L is required for a fixed inner diameter D of the nozzle. Meanwhile, only in the case that the nozzle length L is greater than or equal to the minimum nozzle length 1, a situation that irregular flowing airflow generated due to the change of diameter turns into a stable linearly flowing airflow may be achieved, so that the gas may have a stable flow velocity and a stable airflow direction at the moment of passing through the opening.

According to an embodiment of the present invention, the injection angle $\alpha$ of the central nozzle is 2° to 20°, preferably 4° to 17°, more preferably 5° to 14°. In addition, the injection angle $\alpha$ of the peripheral nozzle is 2° to 20°, preferably 4° to 17°, more preferably 5° to 14°.

According to an embodiment of the present invention, the length of the central nozzle is 80-300 mm, preferably 100-270 mm, and more preferably 120-240 mm.

According to an embodiment of the present invention, the length of the peripheral nozzle is 80-300 mm, preferably 100-270 mm, and more preferably 120-240 mm.

The inventors of the present application have found through a large number of calculations and experiments that, under the most preferable conditions, an optimal flow effect may be obtained at an injection angle $\alpha$ between 5° and 14° and a nozzle length of 120-240 mm.

According to an embodiment of the present invention, the aperture diameter D1 of the central opening is the same as or different from the aperture diameter D1' of the peripheral opening. Preferably, the aperture diameter D1 is larger than the aperture diameter D1'. Particularly, where 1.10 ≥ D1/D1' > 1.00, the deterioration of fluidization quality due to the wall effect can be effectively improved. More preferably, the ratio D1/D1' is set to satisfy 1.08 ≥ D1/D1' > 1.00, and still more preferably, the ratio D1/D1' is set to satisfy 1.06 ≥ D1/D1' > 1.01. Where D1/D1' is greater than 1.10, the flow rectification effect of the nozzle may be reduced.

According to an embodiment of the present invention, where an aperture diameter of the central orifice is designated as d (expressed in a unit of mm), an aperture diameter of the peripheral orifice is designated as d' (expressed in a unit of mm), an aperture diameter of the central opening is designated as D1 (expressed in a unit of mm), and an aperture diameter of the peripheral opening is designated as D1' (expressed in a unit of mm), (d'/D1')/(d/D1) ≥1, preferably (d'/D1')/(d/D1) = 1-1.25, (d'/D1')/(d/D1) = 1-1.20, or (d'/D1')/(d/D1) = 1.01-1.10.

According to an embodiment of the present invention, the inner diameter D2 of each of the central nozzles is the same as or different from each other, preferably the same as each other, and is independently 6-50 mm, preferably 10-47 mm, more preferably 12-44 mm.

According to an embodiment of the present invention, the inner diameter D2' of each of the peripheral nozzles is the same as or different from each other, preferably the same as each other, and is independently 5-48 mm, preferably 9-45 mm, more preferably 11-42 mm.

According to an embodiment of the present invention, the inner diameter D2 of the central nozzle is the same as or different from the inner diameter D2' of the peripheral nozzle. Preferably, the inner diameter D2 is larger than the inner diameter D2', more preferably D2/D2' satisfies the relation 1.10 ≥ D2/D2' > 1.00, preferably 1.08 ≥ D2/D2' > 1.00, more preferably 1.06 ≥ D2/D2' > 1.01.

According to an embodiment of the present invention, it also relates to a fluidizing device, particularly a fluidized bed reactor, more particularly an ammonia oxidation fluidized bed reactor. The fluidizing device comprises at least a housing, a fluidizing device chamber defined by the housing, and a gas distribution plate disposed in the fluidizing device chamber. Here, the gas distribution plate is a gas distribution plate according to any of the preceding aspects of the present invention.

According to an embodiment of the present invention, the manner for fixing the gas distribution plate in the fluidizing device is not particularly limited, and any connection manner conventionally used in the art may be adopted.

According to an embodiment of the present invention, the inner chamber of the fluidizing device has a bed of solid particles, particularly catalyst particles, more particularly ammonia oxidation catalyst particles. Here, the ammonia oxidation catalyst may be any ammonia oxidation catalyst conventionally known in the art, and is not particularly limited.

According to an embodiment of the present invention, it also relates to an oxidation or ammoxidation process, comprising the step of subjecting a feedstock to oxidation or ammoxidation reaction with an oxidizing gas to produce an oxidation or ammoxidation product. Here, specific examples of the feedstock include garbage and hydrocarbon feedstock, particularly $C_{2-8}$ olefins or propylene. As the oxidizing gas, air or oxygen may be particularly mentioned. As the oxidation product or ammoxidation product, propylene oxide or acrylonitrile may be particularly mentioned. In addition, the reaction process is carried out using the gas distribution plate according to any of the preceding aspects of the present invention as a gas distribution plate for the oxidizing gas, or in the fluidized bed reactor according to any of the preceding aspects of the present invention.

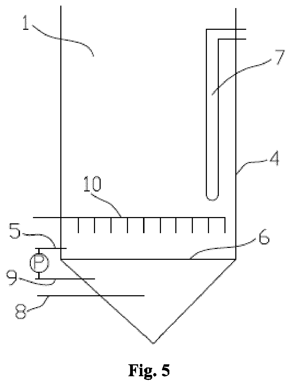

The inventors of the present invention have found that the pressure drop $\Delta P_d$ of the gas distribution plate is an important parameter when the fluidizing apparatus is in operation, particularly when the reactor is used for the oxidation of propylene and ammonia in an acrylonitrile fluidized bed reactor. A good design of the pressure drop of the gas distribution plate can ensure that each nozzle of the gas distribution plate is supplied with the same gas flow rate, namely the gas flow rate per unit cross section of the device is the same. During the process of the gas passing through the orifice, a local pressure loss, that is the pressure difference between the position marked 5 and the position marked 9 in Fig. 5, will be generated, and said pressure difference is the pressure drop $\Delta P_d$ of the gas distribution plate. The greater the pressure drop $\Delta P_d$ of the distribution plate in the fluidizing device, the more uniform the gas distribution.

The inventors of the present invention have also found that there is a correlation between the pressure drop $\Delta P_d$ of the gas distribution plate, the space between the openings, the velocity of gas passing through the opening, and the aperture diameter of the orifice. For fluidizing devices such as acrylonitrile fluidized bed reactors of the same size, if the spaces between openings are the same, the higher the pressure drop $\Delta P_d$ of the gas distribution plate, the higher the velocity of gas passing through the opening, and the smaller the orifice diameter needed. However, the orifice diameter cannot be set too small. Otherwise, on the one hand, abrasion of the orifice may be aggravated due to the high velocity of gas passing through the opening, and on the other hand, the orifice may be easily blocked by foreign matters. Similarly, if the orifice diameters are the same, for a device with a higher pressure drop $\Delta P_d$ of the gas distribution plate, the velocity of gas passing through the opening is higher, and a larger space between openings is needed. Again, the space between openings cannot be infinitely great or infinitely small, but should be correlated to the size of the bubbles generated at a position slightly above the distribution plate.

The inventors of the present invention have further found that the pressure drop $\Delta P_d$ of existing gas distribution plate is typically designed to be 60% of the pressure drop of the bed. But, as the production scale grows continuously, the diameter of the fluidizing device, such as an acrylonitrile fluidized bed reactor, also increases. For example, for an acrylonitrile fluidized bed reactor having a diameter greater than 8.5 meters, if a design parameter of 60% of the pressure drop of the bed is adopted, a larger orifice diameter or a smaller space between openings will be required, as compared to the case where a design parameter of greater than 60% of the pressure drop of the bed. A large orifice diameter is more likely to cause the catalyst to fall into the cone of the reactor without being utilized, which is undesired; alternatively, if the space between openings is small, the number of the openings will be large, and the number of air nozzles will increase, and the number of the branch pipes and nozzles of the propylene-ammonia distributor corresponding to the air nozzles one by one will increase, which will result in an increase in the cost of the equipment. According to the present invention, when the factors of orifice diameter, space between openings and velocity of gas passing through the opening are considered comprehensively, it is appropriate to set the pressure drop $\Delta P_d$ of the gas distribution plate to be 62-120% of the pressure drop $\Delta P_b$ of the bed, preferably 65-115% of the pressure drop $\Delta P_b$ of the bed, and more preferably 68-110% of the pressure drop $\Delta P_b$ of the bed, so that the object of providing the same gas flow rate per unit cross section of the device can be better achieved.

According to a preferred embodiment of the present invention, the gas passing through the gas distribution plate is subjected to the same conditions of temperature, pressure, etc. in the fluidizing device such as acrylonitrile fluidized-bed reactor. In order to control the pressure drop $\Delta P_d$ of the gas distribution plate within the preferred range, the above parameters corresponding to $\Delta P_d$ are typically selected as follows: $P_d$ is designed to be 62-120% of the pressure drop $\Delta P_b$ of the bed, the aperture diameter of the orifice at the lower end of the nozzle of the gas distribution plate is 5-20 mm, and the space between the opening of the gas distribution plate is 100-300 mm. Preferably, $P_d$ is designed to be 65-115% of the pressure drop $\Delta P_b$ of the bed, the aperture diameter of the orifice is 7-18 mm, and the distance between the openings is 125-285 mm. More preferably, $P_d$ is designed to be 68-110% of the pressure drop $\Delta P_b$ of the bed, the aperture diameter of the orifice is 10-16 mm and the distance between the openings is 150-270 mm. Meanwhile, where $P_d$ is in the above range, abrasion of the orifice caused by a high velocity of gas passing through the openings can be avoided.

According to an embodiment of the present invention, the ammoxidation process may be performed in any manner and by any method conventionally known in the art, and such information is known to those skilled in the art, of which the detailed description is omitted herein. Nevertheless, specific examples of the conditions for the reaction process include: a molar ratio of propylene to ammonia to air (calculated on the basis of molecular oxygen) of typically 1 : 1.1-1.3 : 1.8-2.0, a reaction temperature of typically 420-440 °C, a reaction pressure (gauge pressure) of typically 0.03-0.14 MPa, and a weight hourly space velocity of typically 0.04-0.10 $h^{-1}$.

**Examples**

The present application will be described in further detail below with reference to examples and comparative examples, but the present application is not limited to the following examples.

In the following examples and comparative examples, the single-pass yield of acrylonitrile and propylene conversion can be calculated according to the following equations:

$$\text{Single-pass yield of acrylonitrile: AN\%} = C_{AN}/\Sigma C * 100$$

$$\text{Propylene conversion: Cc}_3\% = (1 - Cc_{3out}/Cc_{3in}) * 100$$

wherein:

$C_{AN}$: molar amount (mol) of carbon contained in AN in the gas at the outlet of the reactor
$\Sigma C$: total molar amount (mol) of carbon in the gas at the outlet of the reactor
$Cc_{3out}$: molar amount (mol) of carbon contained in $C_3$ in the gas at the outlet of the reactor
$Cc_{3in}$: molar amount (mol) of carbon contained in $C_3$ in the gas at the inlet of the reactor.

Comparative Example 1

As shown in Fig. 5, the fluidized bed reactor had a diameter of 9.0 m, a propylene feed rate of 9500 NM$^3$/h, a

reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, and the inner diameter D2 was 36 mm.

The number of peripheral openings was 20.5 per square meter of the peripheral region, the aperture diameters D1' of peripheral openings were 36 mm respectively, the space between adjacent peripheral openings was 197 mm, the aperture diameters d' of peripheral orifices were 14.3 mm respectively, and the inner diameter D2' was 30 mm.

D1/D1' was 1.17, d/d' was 1, and (d'/D1')/(d/D1) was 1.17.

Gas phase compositions in the central region, in the wall region and at the outlet of the reactor were taken separately and the results are shown in Table 1.

Comparative Example 2

As shown in Fig. 5, the fluidized bed reactor had a diameter of 9.0 m, a propylene feed rate of 9500 NM³/h, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, and the inner diameter D2 was 36 mm.

The number of peripheral openings was 20.5 per square meter of the peripheral region, the aperture diameters D1' of peripheral openings were 42 mm respectively, the space between adjacent peripheral openings was 197 mm, the aperture diameters d' of peripheral orifices were 14.3 mm respectively, and the inner diameter D2' was 36 mm.

D1/D1' was 1.00, D/D' was 1, and (d'/D1')/(d/D1) was 1.00.

Gas phase compositions in the central region, in the wall region and at the outlet of the reactor were taken separately and the results are shown in Table 1.

Example 1

As shown in Fig. 5, the fluidized bed reactor had a diameter of 9.0 m, a propylene feed rate of 9500 NM³/h, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, and the inner diameter D2 was 36 mm. The velocity of gas passing through the central opening was 10.6 m/s.

The number of peripheral openings was 20.5 per square meter of the peripheral region, the aperture diameters D1' of peripheral openings were 40 mm respectively, the space between adjacent peripheral openings was 197 mm, the aperture diameters d' of peripheral orifices were 14.3 mm respectively, the inner diameter D2' was 34 mm, and the velocity of gas passing through the peripheral opening was 11.8 m/s.

D1/D1' was 1.05, d/d' was 1, and (d'/D1')/(d/D1) was 1.05.

Gas phase compositions in the central region, in the wall region and at the outlet of the reactor were taken separately and the results are shown in Table 1.

Example 2

As shown in Fig. 5, the fluidized bed reactor had a diameter of 9.0 m, a propylene feed rate of 9500 NM³/h, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, and the inner diameter D2 was 36 mm.

The number of peripheral openings was 20.5 per square meter of the peripheral region, the aperture diameters D1' of peripheral openings were 39 mm respectively, the space between adjacent peripheral openings was 197 mm, the aperture diameters d' of peripheral orifices were 14.3 mm respectively, and the inner diameter D2' was 35 mm.

D1/D1' was 1.08, d/d' was 1, and (d'/D1')/(d/D1) was 1.08.

Gas phase compositions in the central region, in the wall region and at the outlet of the reactor were taken separately and the results are shown in Table 1.

Example 3

As shown in Fig. 5, the fluidized bed reactor had a diameter of 9.0 m, a propylene feed rate of 9500 $NM^3/h$, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 13 per square meter of the central region, the central openings each had an aperture diameter D1 of 51 mm, and were evenly arranged in the form of a square at a 275 mm space between openings, the central orifices each had an aperture diameter d of 15.1 mm, and the inner diameter D2 was 45 mm.

The number of peripheral openings was 9 per square meter of the peripheral region, the aperture diameters D1' of peripheral openings were 48 mm respectively, the space between adjacent peripheral openings was 275 mm, the aperture diameters d' of peripheral orifices were 15.1 mm respectively, and the inner diameter D2' was 42 mm.

D1/D1' was 1.06, d/d' was 1, and (d'/D1')/(d/D1) was 1.06.

Gas phase compositions in the central region, in the wall region and at the outlet of the reactor were taken separately and the results are shown in Table 1.

Example 4

As shown in Fig. 5, the fluidized bed reactor had a diameter of 9.0 m, a propylene feed rate of 9500 $NM^3/h$, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 118 per square meter of the central region, the central openings each had an aperture diameter D1 of 25 mm, and were evenly arranged in the form of a triangle at a 95 mm space between openings, the central orifices each had an aperture diameter D of 9.6 mm, and the inner diameter D2 was 20 mm.

The number of peripheral openings was 84 per square meter of the peripheral region, the aperture diameters D1' of peripheral openings were 24 mm respectively, the space between adjacent peripheral openings was 95 mm, the aperture diameters d' of peripheral orifices were 9.6 mm respectively, and the inner diameter D2' was 19 mm.

D1/D1' was 1.04, d/d' was 1, and (d'/D1')/(d/D1) was 1.04.

Gas phase compositions in the central region, in the wall region and at the outlet of the reactor were taken separately and the results are shown in Table 1.

Example 5

As shown in Fig. 5, the fluidized bed reactor had a diameter of 9.0 m, a propylene feed rate of 9500 $NM^3/h$, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, and the inner diameter D2 was 36 mm.

The number of peripheral openings was 20.5 per square meter of the peripheral region, the aperture diameters D1' of peripheral openings were 40 mm respectively, the space between adjacent peripheral openings was 197 mm, the aperture diameters d' of peripheral orifices were 14.1 mm respectively, and the inner diameter D2' was 34 mm.

D1/D1' was 1.05, d/d' was 1.01, and (d'/D1')/(d/D1) was 1.04.

Gas phase compositions in the central region, in the wall region and at the outlet of the reactor were taken separately and the results are shown in Table 1.

Example 6

As shown in Fig. 5, the fluidized bed reactor had a diameter of 9.0 m, a propylene feed rate of 9500 $NM^3/h$, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, and the inner diameter D2 was 36 mm.

The number of peripheral openings was 20.5 per square meter of the peripheral region, the aperture diameters D1' of peripheral openings were 39 mm respectively, the space between adjacent peripheral openings was 197 mm, the aperture diameters d' of peripheral orifices were 12.7 mm respectively, and the inner diameter D2' was 33 mm.

D1/D1' was 1.08, d/d' was 1.13, and (d'/D1')/(d/D1) was 0.96.

Gas phase compositions in the central region, in the wall region and at the outlet of the reactor were taken separately and the results are shown in Table 1.

Example 7

As shown in Fig. 5, the fluidized bed reactor had a diameter of 9.0 m, a propylene feed rate of 9500 NM$^3$/h, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, and the inner diameter D2 was 36 mm.

The number of peripheral openings was 20.5 per square meter of the peripheral region, the aperture diameters D1' of peripheral openings were 40 mm respectively, the space between adjacent peripheral openings was 197 mm, the aperture diameters d' of peripheral orifices were 15.2 mm respectively, and the inner diameter D2' was 34 mm.

D1/D1' was 1.05, d/D' was 0.94, and (d'/D1')/(d/D1) was 1.12.

Gas phase compositions in the central region, in the wall region and at the outlet of the reactor were taken separately and the results are shown in Table 1.

Example 8

As shown in Fig. 5, the fluidized bed reactor had a diameter of 9.0 m, a propylene feed rate of 9500 NM$^3$/h, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, the inner diameter D2 was 36 mm, and the injection angle $\alpha$ of the nozzle was 12°.

The number of the peripheral openings was 20.5 per square meter of the peripheral region, the aperture diameters D1' of peripheral openings were 40 mm respectively, the space between adjacent peripheral openings was 197 mm, the aperture diameters d' of peripheral orifices were 14.3 mm respectively, the inner diameter D2' was 34 mm, and the injection angle $\alpha$ of the nozzle was 12°.

D1/D1' was 1.05, d/d' was 1, and (d'/D1')/(d/D1) was 1.05.

Gas phase compositions in the central region, in the wall region and at the outlet of the reactor were taken separately and the results are shown in Table 1.

Example 9

As shown in Fig. 5, the fluidized bed reactor had a diameter of 9.0 m, a propylene feed rate of 9500 NM$^3$/h, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, the inner diameter D2 was 36 mm, and the injection angle $\alpha$ of the nozzle was 3°.

The number of the peripheral openings was 20.5 per square meter of the peripheral region, the aperture diameters D1' of peripheral openings were 40 mm respectively, the space between adjacent peripheral openings was 197 mm, the aperture diameters d' of peripheral orifices were 14.3 mm respectively, the inner diameter D2' was 34 mm, and the injection angle $\alpha$ of the nozzle was 3°.

D1/D1' was 1.05, d/d' was 1, and (d'/D1')/(d/D1) was 1.05.

Gas phase compositions in the central region, in the wall region and at the outlet of the reactor were taken separately and the results are shown in Table 1.

Due to the small injection angle, the nozzle length is 687 mm, and the device can be implemented, but is not economical.

Example 10

As shown in Fig. 5, the fluidized bed reactor had a diameter of 9.0 m, a propylene feed rate of 9500 NM$^3$/h, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, the inner diameter D2 was 36 mm, and the injection angle $\alpha$ of the nozzle was 25°.

The number of the peripheral openings was 20.5 per square meter of the peripheral region, the aperture diameters D1' of peripheral openings were 40 mm respectively, the space between adjacent peripheral openings was 197 mm, the aperture diameters d' of peripheral orifices were 14.3 mm respectively, the inner diameter D2' was 34 mm, and the injection angle $\alpha$ of the nozzle was 25°.

D1/D1' was 1.05, d/d' was 1, and (d'/D1')/(d/D1) was 1.05.

Gas phase compositions in the central region, in the wall region and at the outlet of the reactor were taken separately and the results are shown in Table 1.

Example 11

As shown in Fig. 5, the fluidized bed reactor had a diameter of 9.0 m, a propylene feed rate of 9500 NM$^3$/h, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at 197 mm space between openings, the central orifices each had an aperture diameter d of 14.3 mm, and the inner diameter D2 was 36 mm.

The number of peripheral openings was 20.5 per square meter of the peripheral region, the aperture diameters D1' of peripheral openings were 41 mm respectively, the space between adjacent peripheral openings was 197 mm, the aperture diameters d' of peripheral orifices were 12.7 mm respectively, and the inner diameter D2' was 35 mm.

D1/D1' was 1.02, d/d' was 1.13, and (d'/D1')/(d/D1) was 0.91.

Gas phase compositions in the central region, in the wall region and at the outlet of the reactor were taken separately and the results are shown in Table 1.

Example 12

As shown in Fig. 5, the fluidized bed reactor had a diameter of 9.0 m, a propylene feed rate of 9500 NM$^3$/h, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.3 mm, and the inner diameter D2 was 36 mm.

The number of peripheral openings was 20.5 per square meter of the peripheral region, the aperture diameters D1' of peripheral openings were 40 mm respectively, the space between adjacent peripheral openings was 197 mm, the aperture diameters d' of peripheral orifices were 14.3 mm respectively, and the inner diameter D2' was 34 mm.

The pressure drop $\Delta P_d$ of the air distribution plate was measured at a full capacity of the device, and was 88% of the pressure drop $\Delta P_b$ of the bed.

D1/D1' was 1.05, d/d' was 1, and (d'/D1')/(d/D1) was 1.05.

Gas phase compositions in the central region, in the wall region and at the outlet of the reactor were taken separately and the results are shown in Table 1.

Example 13

As shown in Fig. 5, the fluidized bed reactor had a diameter of 9.0 m, a propylene feed rate of 9500 $NM^3/h$, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.9 mm, and the inner diameter D2 was 36 mm.

The number of peripheral openings was 20.5 per square meter of the peripheral region, the aperture diameters D1' of peripheral openings were 40 mm respectively, the space between adjacent peripheral openings was 197 mm, the aperture diameters d' of peripheral orifices were 14.9 mm respectively, and the inner diameter D2' was 34 mm.

The pressure drop $\Delta P_d$ of the air distribution plate was measured at a full capacity of the device, and was 55% of the pressure drop $\Delta P_b$ of the bed.

D1/D1' was 1.05, d/d' was 1, and (d'/D1')/(d/D1) was 1.05.

Gas phase compositions in the central region, in the wall region and at the outlet of the reactor were taken separately and the results are shown in Table 1.

Example 14

As shown in Fig. 5, the fluidized bed reactor had a diameter of 9.0 m, a propylene feed rate of 9500 $NM^3/h$, a reaction temperature of 430 °C, a reaction pressure of 0.04 MPa, and a propylene : ammonia : air ratio of 1 : 1.2 : 9.6.

The air distribution plate 6 was a circular metal plate with a diameter of 9.0 m, a thickness of 16 mm and a radius r of the central region of 7.92 m.

The number of central openings was 29 per square meter of the central region, the central openings each had an aperture diameter D1 of 42 mm, and were evenly arranged in the form of a square at a 197 mm space between openings, the central orifices each had an aperture diameter d of 14.6 mm, and the inner diameter D2 was 36 mm.

The number of peripheral openings was 20.5 per square meter of the peripheral region, the aperture diameters D1' of peripheral openings were 40 mm respectively, the space between adjacent peripheral openings was 197 mm, the aperture diameters d' of peripheral orifices were 14.6 mm respectively, and the inner diameter D2' was 34 mm.

The pressure drop $\Delta P_d$ of the air distribution plate was measured at a full capacity of the device, and was 130% of the pressure drop $\Delta P_b$ of the bed.

D1/D1' was 1.05, d/d' was 1, and (d'/D1')/(d/D1) was 1.05.

Gas phase compositions in the central region, in the wall region and at the outlet of the reactor were taken separately and the results are shown in Table 1.

Table 1

| | | Central region | Wall region | Outlet of the reactor | |
|---|---|---|---|---|---|
| | | Unreacted propylene % | | Propylene conversion % | Single-pass yield of acrylonitrile % |
| | Comparative Example 1 | 1.6 | 2.3 | 97.8 | 79.5 |
| | Comparative Example 2 | 1.4 | 2.5 | 97.7 | 79.3 |
| | Example 1 | 1.4 | 1.5 | 98.8 | 81.3 |
| | Example 2 | 1.4 | 1.8 | 98.5 | 80.7 |
| | Example 3 | 1.9 | 2.4 | 97.7 | 79.8 |
| | Example 4 | 1.8 | 2.3 | 97.8 | 79.9 |
| | Example 5 | 1.5 | 1.9 | 98.4 | 81.0 |
| | Example 6 | 1.5 | 2.5 | 98.3 | 80.2 |
| | Example 7 | 1.5 | 2.2 | 98.4 | 80.4 |
| | Example 8 | 1.4 | 1.5 | 98.8 | 81.3 |
| | Example 9 | 1.4 | 1.5 | 98.6 | 81.2 |

(continued)

| | Central region | Wall region | Outlet of the reactor | |
|---|---|---|---|---|
| | Unreacted propylene % | | Propylene conversion % | Single-pass yield of acrylonitrile % |
| Example 10 | 1.8 | 2.5 | 97.7 | 79.8 |
| Example 11 | 1.8 | 2.4 | 98.2 | 80.6 |
| Example 12 | 1.4 | 1.5 | 98.8 | 81.3 |
| Example 13 | 1.8 | 2.3 | 98.1 | 80.5 |
| Example 14 | 1.5 | 1.9 | 98.4 | 81.0 |

As can be seen from Table 1, by using the gas distribution plate of the present invention, the fluidization quality of the catalyst near the reactor wall can be improved and the propylene conversion and the yield of acrylonitrile can be significantly increased.

**Claims**

1. A gas distribution plate (particularly an air distribution plate or an air distribution plate for an ammoxidation fluidized bed reactor), comprising a metal plate (particularly a flat metal plate), openings provided in a central region of the metal plate (referred to as central openings) and openings provided in a peripheral region of the metal plate (referred to as peripheral openings), wherein a ratio $D1/D1'$ of an aperture diameter $D1$ (expressed in a unit of mm) of the central opening to an aperture diameter $D1'$ (expressed in a unit of mm) of the peripheral opening satisfies the relation $1.10 \geq D1/D1' > 1.00$, preferably $1.08 \geq D1/D1' > 1.00$, and more preferably $1.06 \geq D1/D1' \geq 1.01$.

2. The gas distribution plate according to claim 1, wherein the aperture diameter $D1$ of each of the central openings is the same as or different from each other (preferably the same as each other) and is independently 16-60 mm, preferably 20-56 mm, more preferably 22-52 mm, and/or the aperture diameter $D1'$ of each of the peripheral openings is the same as or different from each other (preferably the same as each other) and is independently 15-58 mm, preferably 19-54 mm, more preferably 21-50 mm.

3. The gas distribution plate according to claim 1, wherein the number of central openings is from 16 to 100 (preferably from 17 to 64, more preferably from 18 to 44) per square meter of the central region, and/or the number of peripheral openings is from 2 to 50 (preferably from 3 to 44, more preferably from 4 to 25) per square meter of the peripheral region, and/or the number of central openings is from 70% to 99% (preferably from 75% to 98%, more preferably from 80% to 95%) of the total number of openings in the metal plate.

4. The gas distribution plate according to claim 1, wherein the number of central openings per unit area of the central region is substantially the same.

5. The gas distribution plate according to claim 1, wherein the central openings and/or the peripheral openings are arranged substantially in the form of a square, an equilateral triangle, an equilateral rhombus, or concentric circles, preferably substantially in the form of a square or an equilateral triangle.

6. The gas distribution plate according to claim 1, wherein the distances between any two adjacent central openings are the same as or different from each other (preferably the same as each other), and are each independently 100-300 mm, preferably 125-285 mm, more preferably 150-270 mm, and/or the distances between any two adjacent peripheral openings are the same as or different from each other (preferably the same as each other), and are each independently 100-300 mm, preferably 125-285 mm, more preferably 150-270 mm.

7. The gas distribution plate according to claim 1, wherein the metal plate has a substantially circular shape, the circle having a diameter of 5 to 29 m, preferably 7 to 20 m, and a thickness of 5 to 40 mm, preferably 10 to 35 mm.

8. The gas distribution plate according to claim 1, where a straight-line distance between any point on the outer periphery of the metal plate and a central point of the metal plate is designated as R (particularly, a radius), a region surrounded by all points on the metal plate at a straight-line distance r from the central point is referred to as the central region, and a region between the central region and the outer periphery is referred to as the peripheral region, the value of r/R is 0.2 to 0.99, preferably 0.5 to 0.9, and more preferably 0.7 to 0.85.

9. The gas distribution plate according to claim 1, wherein at least one (preferably all) of the central openings have a nozzle (referred to as central nozzle), wherein the central nozzle is a hollow tube with a starting end of the central nozzle being inserted into the central opening, perpendicularly connected to the metal plate and coaxial with the central opening, and a tail end of the central nozzle having an orifice (referred to as central orifice), and/or at least one (preferably all) of the peripheral openings have a nozzle (referred to as peripheral nozzle), wherein the peripheral nozzle is a hollow tube with a starting end of the peripheral nozzle being inserted into the peripheral opening, perpendicularly connected to the metal plate and coaxial with the peripheral opening, and a tail end of the peripheral nozzle having an orifice (referred to as peripheral orifice).

10. The gas distribution plate according to claim 9, wherein the aperture diameter d of each of the central orifices is the same as or different from each other (preferably the same as each other) and is independently 5 to 20mm, preferably 7 to 18mm, and more preferably 10 to 16 mm, and/or the aperture diameter d' of each of the peripheral orifices is the same as or different from each other (preferably the same as each other) and is independently 5 to 20 mm, preferably 7 to 18 mm, and more preferably 10 to 16 mm, and/or the aperture diameter d of the central orifice is the same as or different from the aperture diameter d' of the peripheral orifice, and/or d/d' satisfies the relation $1.10 \geq d/d' \geq 1.00$ (preferably $1.04 \geq d/d' \geq 1.00$).

11. The gas distribution plate according to claim 10, wherein the central nozzles and/or the peripheral nozzles have an injection angle $\alpha$ of 2° to 20°, preferably 4° to 17°, more preferably 5° to 14°.

12. The gas distribution plate according to claim 10, wherein the length of the central nozzle and/or the peripheral nozzle is 80-300 mm, preferably 100-270 mm, and more preferably 120-240 mm.

13. The gas distribution plate according to claim 9, where the aperture diameter of the center orifice is designated as d (expressed in a unit of mm), the aperture diameter of the peripheral orifice is designated as d' (expressed in a unit of mm), the aperture diameter of the central opening is designated as D1 (expressed in a unit of mm), and the aperture diameter of the peripheral opening is designate as D1' (expressed in a unit of mm), (d'/D1')/(d/D1) $\geq$ 1, preferably (d'/D1')/(d/D1) = 1-1.25, (d'/D1')/(d/D1) = 1-1.20, or (d'/D1')/(d/D1) = 1.01-1.10.

14. The gas distribution plate according to claim 9, wherein the inner diameter D2 of each of the central nozzles is the same as or different from each other (preferably the same as each other) and is independently 6 to 50 mm, preferably 10 to 47 mm, more preferably 12 to 44 mm, and/or the inner diameter D2' of each of the peripheral nozzles is the same as or different from each other (preferably the same as each other) and is independently 5 to 48 mm, preferably 9 to 45 mm, more preferably 11 to 42 mm.

15. A gas distribution plate, comprising a metal plate, central openings provided in a central region of the metal plate, and peripheral openings provided in a peripheral region of the metal plate, wherein at least one (preferably all) of the central openings have a nozzle (referred to as central nozzle), wherein the central nozzle is a hollow tube with a starting end of the central nozzle being inserted into the central opening, perpendicularly connected to the metal plate and coaxial with the central opening, and a tail end of the central nozzle having an orifice (referred to as central orifice), and at least one (preferably all) of the peripheral openings have a nozzle (referred to as peripheral nozzle), wherein the peripheral nozzle is a hollow tube with a starting end of the peripheral nozzle being inserted into the peripheral opening, perpendicularly connected to the metal plate and coaxial with the peripheral opening, and a tail end of the peripheral nozzle having an orifice (referred to as peripheral orifice), and where an aperture diameter of the central orifice is designated as d (expressed in a unit of mm), an aperture diameter of the peripheral orifice is designated as d' (expressed in a unit of mm), an aperture diameter of the central opening is designated as D1 (expressed in a unit of mm), and an aperture diameter of the peripheral opening is designated as D1' (expressed in a unit of mm), (d'/D1')/(d/D1) $\geq$ 1, preferably (d'/D1')/(d/D1) = 1-1.25, (d'/D1')/(d/D1) = 1-1.20, or (d'/D1')/(d/D1) = 1.01-1.10.

16. A fluidizing device (preferably a fluidized bed reactor), comprising at least a housing, a fluidizing device chamber defined by the housing, and a gas distribution plate disposed in the fluidizing device chamber, wherein the gas distribution plate is a gas distribution plate according to claim 1 or 15.

17. The fluidizing device according to claim 16, wherein the fluidizing device chamber has a bed of solid particles (preferably catalyst particles), and wherein a pressure drop ΔPd (expressed in a unit of MPa) of the gas distribution plate is 62-120%, preferably 65-115%, more preferably 68-110% of a pressure drop ΔPb (expressed in a unit of MPa) of the bed of solid particles.

18. An oxidation or ammoxidation process, comprising the step of subjecting a feedstock (preferably a garbage or hydrocarbon feedstock, more preferably $C_{2-8}$ olefin or propylene) to oxidation or ammoxidation reaction with an oxidizing gas (preferably air or oxygen) to produce an oxidation or ammoxidation product (preferably propylene oxide or acrylonitrile) using a gas distribution plate according to claim 1 or 15 as the oxidizing gas (preferably air or oxygen) distribution plate or in a fluidized bed reactor according to claim 16.

19. A method for distributing a gas, comprising the step of delivering the gas through a gas distribution plate according to claim 1 or 15 from one side thereof to the other, wherein the flow velocity of gas passing through the peripheral opening is not less than the flow velocity of gas passing through the central opening.

**Fig. 1**

**Fig. 2A**                    **Fig. 2B**

**Fig. 3**

**Fig. 4**

**Fig. 5**

## INTERNATIONAL SEARCH REPORT

| International application No. |
| --- |
| **PCT/CN2020/129819** |

| A. | CLASSIFICATION OF SUBJECT MATTER |
| --- | --- |

B01J 8/24(2006.01)i; C07C 253/26(2006.01)i; B01J 8/44(2006.01)i

According to International Patent Classification (IPC) or to both national classification and IPC

| B. | FIELDS SEARCHED |
| --- | --- |

Minimum documentation searched (classification system followed by classification symbols)

B01J8,C07C253

Documentation searched other than minimum documentation to the extent that such documents are included in the fields searched

Electronic data base consulted during the international search (name of data base and, where practicable, search terms used)

DWPI; SIPOABS; CNABS; CNTXT; CNKI: 流化床, 气体, 空气, 气流, 分布, 分配, 孔径, 直径, 尺寸, 边缘, 外缘, 四周, 圆周, 中心, 中央, 壁, 喷嘴, 喷咀, 喷头, 喷射, 喷洒, 孔, 开口, fluidi+, distribut+, dispens+, diameter, size, center, central, inner, peripheral, edge, circumference, wall, nozzle, sparger, opening, hole, perforat+, orifice

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | CN 1060835 A (ASAHI CHEMICAL INDUSTRY CO., LTD.) 06 May 1992 (1992-05-06) page 6 line 9- page 9 last line, embodiments 1-9, figures 1-3 | 1-19 |
| Y | CN 1200457 A (NIPPON OIL CO., LTD.) 02 December 1998 (1998-12-02) claims 1 and 4 | 1-19 |
| Y | CN 1175219 A (HOECHST AKTIENGESELLSCHAFT) 04 March 1998 (1998-03-04) claims 1-4, and figure 1 | 1-19 |
| A | US 4436507 A (FOSTER WHEELER ENERGY CORP) 13 March 1984 (1984-03-13) entire document | 1-19 |
| A | CN 1144713 A (MITSUI PETROCHEMICAL INDUSTRIES LTD.) 12 March 1997 (1997-03-12) entire document | 1-19 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **12 January 2021** | **05 February 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

<div align="center">

**INTERNATIONAL SEARCH REPORT**
Information on patent family members

</div>

| International application No. |
|---|
| **PCT/CN2020/129819** |

| Patent document cited in search report | | | Publication date (day/month/year) | Patent family member(s) | | | Publication date (day/month/year) |
|---|---|---|---|---|---|---|---|
| CN | 1060835 | A | 06 May 1992 | CN | 1041413 | C | 30 December 1998 |
| | | | | WO | 9104961 | A1 | 18 April 1991 |
| | | | | DE | 69007965 | D1 | 11 May 1994 |
| | | | | SU | 1829957 | A3 | 23 July 1993 |
| | | | | JP | H03123767 | A | 27 May 1991 |
| | | | | KR | 940007528 | B1 | 19 August 1994 |
| | | | | ES | 2051025 | T3 | 01 June 1994 |
| | | | | EP | 0446379 | A4 | 11 March 1992 |
| | | | | EP | 0446379 | B1 | 06 April 1994 |
| | | | | EP | 0446379 | A1 | 18 September 1991 |
| | | | | CA | 2042584 | C | 09 November 1993 |
| | | | | JP | H03120247 | A | 22 May 1991 |
| CN | 1200457 | A | 02 December 1998 | JP | H10323553 | A | 08 December 1998 |
| CN | 1175219 | A | 04 March 1998 | PL | 321830 | A1 | 22 December 1997 |
| | | | | SK | 113497 | A3 | 14 January 1998 |
| | | | | DE | 59504023 | D1 | 26 November 1998 |
| | | | | WO | 9626003 | A1 | 29 August 1996 |
| | | | | BR | 9510383 | A | 02 June 1998 |
| | | | | BG | 101745 | A | 29 May 1998 |
| | | | | NO | 973714 | D0 | 12 August 1997 |
| | | | | CZ | 265797 | A3 | 18 February 1998 |
| | | | | ES | 2126296 | T3 | 16 March 1999 |
| | | | | EP | 0810902 | B1 | 21 October 1998 |
| | | | | CZ | 289342 | B6 | 16 January 2002 |
| | | | | PL | 180784 | B1 | 30 April 2001 |
| | | | | HU | 221883 | B1 | 28 February 2003 |
| | | | | CA | 2213446 | A1 | 29 August 1996 |
| | | | | DE | 19505664 | C2 | 12 December 1996 |
| | | | | IN | 188066 | B | 17 August 2002 |
| | | | | CA | 2213446 | C | 29 January 2002 |
| | | | | BG | 62436 | B1 | 30 November 1999 |
| | | | | CZ | 9702657 | A3 | 18 February 1998 |
| | | | | HU | T77918 | A | 28 October 1998 |
| | | | | KR | 19980702341 | A | 15 July 1998 |
| | | | | AU | 2881095 | A | 11 September 1996 |
| | | | | ZA | 961278 | B | 27 August 1996 |
| | | | | JP | H11500062 | A | 06 January 1999 |
| | | | | EP | 0810902 | A1 | 10 December 1997 |
| | | | | SK | 282850 | B6 | 03 December 2002 |
| | | | | RO | 118119 | B1 | 28 February 2003 |
| | | | | AU | 702305 | B2 | 18 February 1999 |
| | | | | DE | 19505664 | A1 | 29 August 1996 |
| | | | | KR | 100368512 | B1 | 26 March 2003 |
| | | | | CN | 1089026 | C | 14 August 2002 |
| | | | | RU | 2157726 | C2 | 20 October 2000 |
| | | | | NO | 973714 | L | 12 August 1997 |
| | | | | MX | 9706276 | A | 29 November 1997 |
| | | | | NO | 973714 | A | 12 August 1997 |
| | | | | ZA | 9601278 | A | 27 August 1996 |
| US | 4436507 | A | 13 March 1984 | None | | | |

Form PCT/ISA/210 (patent family annex) (January 2015)

## INTERNATIONAL SEARCH REPORT
### Information on patent family members

| International application No. |
|---|
| **PCT/CN2020/129819** |

| Patent document cited in search report | | | Publication date (day/month/year) | Patent family member(s) | | | Publication date (day/month/year) |
|---|---|---|---|---|---|---|---|
| CN | 1144713 | A | 12 March 1997 | EP | 0721798 | B1 | 06 March 2002 |
| | | | | DE | 69531374 | D1 | 28 August 2003 |
| | | | | JP | 3497029 | B2 | 16 February 2004 |
| | | | | SG | 41968 | A1 | 15 August 1997 |
| | | | | CA | 2166171 | A1 | 29 June 1996 |
| | | | | EP | 1160008 | A1 | 05 December 2001 |
| | | | | TW | 304892 | B | 11 May 1997 |
| | | | | DE | 69525729 | T2 | 08 August 2002 |
| | | | | KR | 960022582 | A | 18 July 1996 |
| | | | | EP | 0721798 | A2 | 17 July 1996 |
| | | | | MY | 115388 | A | 31 May 2003 |
| | | | | DE | 69531374 | T2 | 15 April 2004 |
| | | | | EP | 0721798 | A3 | 02 January 1997 |
| | | | | KR | 100178073 | B1 | 15 May 1999 |
| | | | | DE | 69525729 | D1 | 11 April 2002 |
| | | | | CN | 1080583 | C | 13 March 2002 |
| | | | | EP | 1160008 | B1 | 23 July 2003 |
| | | | | US | 5753191 | A | 19 May 1998 |
| | | | | JP | H08245709 | A | 24 September 1996 |
| | | | | KR | 0178073 | B1 | 15 May 1999 |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 1265770 A

**Non-patent literature cited in the description**

- *Study on Capacity Expansion and Modification of Acrylonitrile Plant (Contemporary Chemical Industry,* vol. 34 (5